# EUROPEAN PATENT APPLICATION

(11) **EP 4 424 678 A1**
(43) Date of publication of application: **04.09.2024**
(21) Application number: 22885930.2
(22) Date of filing: 25.10.2022
(51) Int. Cl.: C07D 307/46, C07D 307/68

(54) **METHOD AND SYSTEM FOR CONTINUOUSLY PRODUCING 5-HYDROXYMETHYLFURFURAL AND 2,5-FURANDICARBOXYLIC ACID**

(30) Priority: 29.10.2021 CN 202111271775
(71) Applicant: China Petroleum & Chemical Corporation, Beijing 100728 (CN); Sinopec Research Institute of Petroleum Processing Co., Ltd., Beijing 100083 (CN)
(72) Inventor: SUN, Qianhui, Beijing 100083 (CN); CHEN, Gongzhe, Beijing 100083 (CN); ZHENG, Lufan, Beijing 100083 (CN); DU, Zexue, Beijing 100083 (CN); ZONG, Baoning, Beijing 100083 (CN)
(74) Representative: SSM Sandmair
(86) International application number: PCT/CN2022/127316
(87) International publication number: WO 2023/072053

(57) **Abstract**

Disclosed is a process for continuously producing 5-hydroxymethylfurfural and 2,5-furandicarboxylic acid, comprising: (1) in a dual liquid phase reaction medium containing a polar organic solvent and an aqueous halogenated quaternary ammonium salt solution, in the presence of a protonic acid catalyst, subjecting a fructose-based carbohydrate to an intramolecular dehydration reaction to produce an organic phase containing 5-hydroxymethylfurfural; and (2) adding water to the organic phase containing 5-hydroxymethylfurfural obtained in step (1), and in the presence of an oxidation catalyst and oxygen gas, subjecting the 5-hydroxymethylfurfural to an oxidation reaction to produce 2,5-furandicarboxylic acid. The process can continuously produce 5-hydroxymethylfurfural and 2,5-furandicarboxylic acid by starting from fructose-based carbohydrates, avoids the separation and purification of 5-hydroxymethylfurfural during the process of producing 2,5-furandicarboxylic acid, and has the advantages such as simplicity, low generation and emission of three wastes, environmental friendliness, and the like.

## Description

### Technical Field

The present application relates to the preparation of oxygen-containing organic compounds, and specifically to a process and system for the continuous production of 5-hydroxymethylfurfural and 2,5-furandicarboxylic acid.

### Background of Art

Due to the renewable and widely sourced characteristics of biomass, the study on using it as raw material to prepare fuels and fine chemicals through chemical catalysis is currently an important research route for biomass utilization and conversion. Sugar compounds, represented by glucose and fructose, are important components of biomass resources. Starting from sugar compounds, new platform compounds and intermediates are prepared through chemical processes. Through further conversion, they can effectively replace chemical products obtained from fossil resources, thereby effectively alleviating the energy crisis and environmental problems caused by the current large-scale use of fossil resources. In recent years, a product obtained by dehydration of hexose as raw material, 5-hydroxymethylfurfural (HMF), has been considered as an important bio-based furan compound. This compound and its derivatives can be further converted into various fine chemicals, and have potential applications in polymer monomers, furan pharmaceutical intermediates, pesticide chemicals, and other fields. Therefore, developing an efficient process for utilizing sugar compounds to prepare HMF is a key issue for the effective utilization of biological resources. Meanwhile, 2,5-furandicarboxylic acid (FDCA) is obtained by catalytic oxidation of HMF. FDCA is used to synthesize bio-based polymer materials, which can effectively improve their heat resistance and mechanical properties. It is considered as an ideal substitute for petroleum-based monomer terephthalic acid (PTA) and can be widely used in the synthesis of bio-based polymers such as polyester, polyamide, and epoxy resin. Therefore, developing a synthesis process for 2,5-furandicarboxylic acid has important application value and is of significance in the biomass sustainable utilization.

HMF can be prepared from glucose or fructose. Compared to glucose, the fructose route does not require isomerization, and only requires the intramolecular dehydration of fructose under the protonic acid catalysis to obtain the target product. The reaction rate is faster and the product yield is higher. It is currently a hot research topic in the HMF preparation process. Research has shown that in order to improve the production yield of HMF and achieve large-scale production of HMF as soon as possible, it is urgent to establish a dual liquid phase solvent system suitable for the liquid acid catalyst to achieve the HMF continuous production technology. However, common liquid acid catalysts have high solubility in water and organic solvents, making it difficult to separate from reaction media and products. Therefore, while improving the yield and purity of the target product, it is necessary to establish effective processes for the recovery of liquid acid catalysts through appropriate means, and strengthen the effective separation and comprehensive utilization of other by-products and impurities to reduce environmental pressure. In addition, we also need to pay attention to reaction factors other than catalysts and organic solvents, such as the role of salt compounds in improving the HMF yield, breaking the inhibition of existing reaction factors on the HMF yield.

On the other hand, in the process of preparing FDCA by selective oxidation of HMF, due to the low solubility of FDCA in water (<1 g/100 mL water, 100°C), researchers often add alkaline compounds in the reaction process, which generate soluble salt compounds of FDCA with the product FDCA, therefore increasing the one-way processing capacity of the process. However, the salt compounds of FDCA obtained by these processes cannot be directly used in the production of polymers and other processes, and must be subjected to acidification (pH≈1) treatment to convert them back into FDCA products. This undoubtedly increases the complexity of the overall production process of FDCA and reduces its environmental friendliness. CN108779088A discloses a process for preparing FDCA by one-step or two-step oxidation of HMF without introducing alkaline compounds. This process effectively improves the solubility of FDCA by using a mixed solvent of water and organic solvents. The catalyst used in this process is a loaded metal catalyst, wherein the metal component is Pt and Au, and the specific surface area of the support is limited to a range of 20 m²/g to 500 m²/g. Due to the high prices of Pt and Au (about 300-400 yuan/g), the overall process cost is relatively high, making it difficult to apply in practice. CN111039906A achieves the method of preparing FDCA by oxidizing HMF in a mixed solvent of water and organic solvents by doping N elements on the surface of a carbon-based support. However, the process of doping the support with nitrogen in this method is relatively complex, which correspondingly increases the preparation cost of the catalyst and is also difficult to apply in practice.

Therefore, it is urgent to provide a simple, inexpensive, and green process for synthesizing 2,5-furandicarboxylic acid to solve the above-mentioned problems in existing technologies.

It should be noted that the information disclosed in the aforementioned section "Background of Art" is only intended to enhance the understanding of the background of the present application, and therefore it may include information on existing technologies known to those ordinarily skilled in the art.

### Summary of the Invention

One object of the present application is to provide a process and system for the continuous production of 5-hydroxymethylfurfural and 2,5-furandicarboxylic acid. The process can continuously produce 5-hydroxymethylfurfural and 2,5-furandicarboxylic acid by starting from fructose-based carbohydrates, avoids the separation and purification of 5-hydroxymethylfurfural during the process of producing 2,5-furandicarboxylic acid, and has the advantages such as simplicity, low generation and emission of three wastes, environmental friendliness, and the like.

Another object of the present application is to provide a process for producing 2,5-furandicarboxylic acid from 5-hydroxymethylfurfural, which process can avoid the introduction of alkaline compounds in traditional processes and the complex process such as the acidification treatment caused thereby. At the same time, the process can use a Ru-based catalyst loaded on a high specific surface area carbon-containing support as an oxidation catalyst. The preparation method of the catalyst is simple and inexpensive, the reaction process is green and concise, and the FDCA yield is high.

In order to achieve the above obj ects, in one aspect, the present application provides a process for continuously producing 5-hydroxymethylfurfural and 2,5-furandicarboxylic acid, including:
(1) in a dual liquid phase reaction medium containing a polar organic solvent and an aqueous halogenated quaternary ammonium salt solution, in the presence of a protonic acid catalyst, subjecting a fructose-based carbohydrate to an intramolecular dehydration reaction to produce an organic phase containing 5-hydroxymethylfurfural; and
(2) adding water to the organic phase containing 5-hydroxymethylfurfural obtained in step (1), and in the presence of an oxidation catalyst and oxygen gas, subjecting the 5-hydroxymethylfurfural to an oxidation reaction to produce 2,5-furandicarboxylic acid,

wherein, the protonic acid catalyst is selected from hydrochloric acid, sulfuric acid, sulfonic acid, phosphoric acid, sulfamic acid, or combinations thereof;
the oxidation catalyst comprises a support and an active metal component loaded on the support, the active metal component contains a precious metal selected from Ru, Pt, Pd, Au or combinations thereof.

In another aspect, the present application provides a process for producing 2,5-furandicarboxylic acid, comprising the following steps:
in a mixed solvent containing an organic solvent and water, in the presence of an oxidation catalyst and oxygen gas, subjecting 5-hydroxymethylfurfural to an oxidation reaction to produce 2,5-furandicarboxylic acid;
wherein the organic solvent is an aprotic polar organic solvent miscible with water, the oxidation catalyst comprises a support and an active metal component loaded on the support, wherein the support is an activated carbon having a specific surface area of 1000-2000 m²/g, and the active metal component contains Ru.

In yet another aspect, the present application provides a reaction system for continuously producing 5-hydroxymethylfurfural and 2,5-furandicarboxylic acid, which comprises a dehydration reaction unit, a phase separation unit, and an oxidation reaction unit, wherein the dehydration reaction unit has an inlet and an outlet, the phase separation unit has an inlet, an aqueous phase outlet and an organic phase outlet, the oxidation reaction unit has an inlet and an outlet, the outlet of the dehydration reaction unit is communicated with the inlet of the phase separation unit, the aqueous phase outlet of the phase separation unit is communicated with the inlet of the dehydration reaction unit, and the organic phase outlet of the phase separation unit is communicated with the inlet of the oxidation reaction unit,
wherein a fructose-based carbohydrate is subjected to a dehydration reaction in a dual liquid phase reaction medium containing a polar organic solvent and an aqueous halogenated quaternary ammonium salt solution in the presence of a protonic acid catalyst in the dehydration reaction unit to produce a two-phase stream containing 5-hydroxymethylfurfural, the two-phase stream is subjected to a phase separation in the phase separation unit to produce an aqueous phase containing the acid catalyst and an organic phase containing 5-hydroxymethylfurfural, the aqueous phase containing the acid catalyst is fed back to the dehydration reaction unit, and the organic phase containing 5-hydroxymethylfurfural is subjected to an oxidation reaction in the oxidation reaction unit to produce a stream containing 2,5-furandicarboxylic acid.

The process and system for continuously producing 5-hydroxymethylfurfural and 2,5-furandicarboxylic acid of the present application have one or more of the following advantages:
1. The catalysts and organic solvents used are conducive to the large-scale production of 5-hydroxymethylfurfural (HMF);
2. By regulating the solubility of HMF and the acid catalyst in the dual phase solution, HMF and the acid catalyst are retained respectively in the organic and aqueous phases after the dehydration reaction, solving the difficult problem of separating the liquid acid catalyst from the reaction medium and the target product, greatly simplifying the separation operation of the catalyst, and achieving the continuous operation of the reaction;
3. During the dehydration reaction, the in-situ separation of HMF and the acid catalyst is achieved, reducing the acid amount of HMF in the organic phase and avoiding the decomposition of HMF caused by the presence of the acid catalyst during the high-temperature in-situ extraction of HMF;
4. Using the organic phase containing HMF as raw material directly to produce 2,5-furandicarboxylic acid (FDCA) avoids the intermediate separation and purification processes, simplifying the production process of FDCA;
5. In the oxidation reaction for preparing FDCA, a mixed solvent formed from the organic solution and water is used to increase the solubility of FDCA, avoid the introduction of alkaline compounds, and also avoid the generation of FDCA salt products. A solution containing FDCA is directly obtained, and after separation and purification of the solution, FDCA can be obtained, greatly simplifying the process flow and at the same time, avoiding the situation where the acidification process leads to the generation of a large amount of waste acid and wastewater; and
6. A Ru-based catalyst loaded on the carbon-containing material with a high specific surface area can be used in the oxidation reaction step of the process of the present application. The preparation process of the catalyst is simple, more inexpensive, and convenient for scaling up the production with good industrial application prospect.

### Brief Description of the Drawing

The accompanying drawing, constituting a part of the present description, is intended to provide a further understanding of the present application, and should not be considered to be limiting. The present application can be interpreted with reference to the drawing in combination with the detailed description hereinbelow. In the drawing:

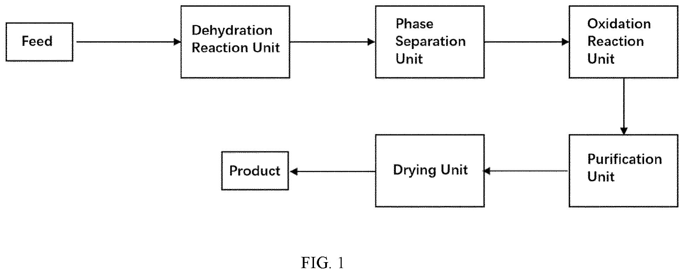

Figure 1 shows a schematic flowchart of a preferable embodiment of the reaction system of the present application.

### Detailed description of the Invention

The following provides a detailed explanation of specific embodiments of the present application in conjunction with the accompanying drawing. It should be understood that the specific embodiments described herein are only intended to illustrate and explain the present application, and are not intended to limit the present application.

Any specific numerical value disclosed herein (including the endpoints of a numerical range) is not limited to the precise value of the numerical value, but is to be understood to further cover values close to the precise value, e.g. all possible values within ±5% of the precise value. Moreover, for the disclosed numerical ranges, one or more new numerical ranges can be obtained by any combination between the endpoint values of the ranges, between one endpoint value of the ranges and one specific point value within the ranges, and between any two specific point values of the ranges. These new numerical ranges shall also be deemed to be specifically disclosed herein.

Unless otherwise stated, the terms used herein have the same meanings as commonly understood by those skilled in the art. If a term is defined herein and its definition is different from the common understanding in the art, the definition herein shall prevail.

In the present application, the term "sulfonic acid" refers to a class of strongly acidic organic compounds formed by connecting a sulfonic group (-SO₃H) with a hydrocarbyl, with the general formula R-SO₃H, where R is the hydrocarbyl, such as alkyl or aryl, especially C₁₋₁₂ alkyl or C₆₋₁₂ aryl, and the alkyl and aryl groups can also optionally have a substituent group, such as hydroxyl.

In the present application, the term "low boiling point polar organic solvent" refers to a polar organic solvent having a boiling point close to or lower than that of water (for example having a boiling point below 110°C at atmospheric pressure).

In the present application, the term "halogenated quaternary ammonium salt" has a commonly understood meaning in the art, and particularly refers to a compound with the general formula R₄NX, where each group R is independently a hydrocarbyl, such as C₁₋₁₂ hydrocarbyl, and X is halogen, such as fluorine, chlorine, bromine, or iodine. Optionally, the hydrocarbyl R may also have a substituent, such as hydroxyl and halogen.

In the present application, the term "hydrocarbyl" has a commonly understood meaning in the art, and specifically refers to a group formed by removing a hydrogen atom from a hydrocarbon compound, which can be saturated or unsaturated, including various aliphatic, alicyclic, and aromatic hydrocarbyls, such as methyl, ethyl, propyl, butyl, allyl, cyclohexyl, and phenyl.

In the present application, unless expressly stated, any issues or matters not mentioned shall directly apply to those known in the art without any change. Moreover, any embodiment described herein can be freely combined with one or more other embodiments described herein, and the technical solutions or technical ideas thus formed shall be regarded as a part of the original disclosure or content of the present application and shall not be considered to be new matter that has not been disclosed or expected herein, unless those skilled in the art believe that the combination is obviously unreasonable.

All patent literatures and non-patent literatures including but not limited to textbooks and journal articles mentioned herein are incorporated by reference in their entirety.

As mentioned above, in a first aspect, the present application provides a process for continuously producing 5-hydroxymethylfurfural and 2,5-furandicarboxylic acid, comprising:
(1) in a dual liquid phase reaction medium containing a polar organic solvent and an aqueous halogenated quaternary ammonium salt solution, in the presence of a protonic acid catalyst, subjecting a fructose-based carbohydrate to an intramolecular dehydration reaction to produce an organic phase containing 5-hydroxymethylfurfural; and
(2) adding water to the organic phase containing 5-hydroxymethylfurfural obtained in step (1), and in the presence of an oxidation catalyst and oxygen gas, subjecting the 5-hydroxymethylfurfural to an oxidation reaction to produce 2,5-furandicarboxylic acid.

In a preferable embodiment, in step (1), the fructose-based carbohydrate is selected from purified fructose, crude fructose, polyfructose, fructose syrup, fructose dextrose syrup, or combinations thereof.

In a preferable embodiment, in step (1), the halogenated quaternary ammonium salt is selected from a halogenated quaternary ammonium salt having an unsubstituted C₁₋₁₂ hydrocarbyl, a halogenated quaternary ammonium salt having a hydroxy or halogen-substituted C₁₋₁₂ hydrocarbyl or combinations thereof, more preferably selected from an unsubstituted C₁₋₆ hydrocarbyl trimethylammonium halide, a hydroxy or halogen-substituted C₁₋₆ hydrocarbyl trimethylammonium halide or combinations thereof, still further preferably selected from an unsubstituted C₁₋₄ hydrocarbyl trimethylammonium chloride, an unsubstituted C₁₋₄ hydrocarbyl trimethylammonium bromide, a hydroxy or halogen-substituted C₁₋₄ hydrocarbyl trimethylammonium chloride, a hydroxy or halogen-substituted C₁₋₄ hydrocarbyl trimethyl ammonium bromide, or combinations thereof. Particularly preferably, the halogenated quaternary ammonium salt is selected from tetramethylammonium chloride, tetramethylammonium bromide, choline chloride, chlorocholine chloride (chlormequat chloride), allyltrimethylammonium chloride, butyltrimethylammonium chloride, or combinations thereof, still further preferably selected from tetramethylammonium chloride, choline chloride, chlorocholine chloride or combinations thereof.

In a preferable embodiment, in step (1), the polar organic solvent is an aprotic polar organic solvent miscible with water, preferably selected from acetone, tetrahydrofuran, 1,4-dioxane, acetonitrile, or combinations thereof, more preferably 1,4-dioxane.

The protonic acid catalyst suitable for the process of the present application can be an inorganic acid, such as hydrochloric acid, sulfuric acid, phosphoric acid, sulfamic acid, and nitric acid; or an organic acid, such as aliphatic carboxylic acid (such as acetic acid and propionic acid), sulfonic acid, and sulfinic acid; preferably selected from hydrochloric acid, sulfuric acid, sulfonic acid, phosphoric acid, sulfamic acid, or combinations thereof, more preferably selected from sulfamic acid, sulfuric acid and sulfonic acid, particularly preferably selected from sulfuric acid and sulfonic acid. In the process of the present application, in the case that sulfamic acid, sulfuric acid and sulfonic acid are used as the protonic acid catalyst, a higher catalyst recovery rate can be achieved. In a particularly preferable embodiment, the sulfonic acid is selected from benzenesulfonic acid, methylsulfonic acid or combinations thereof, more preferably methylsulfonic acid.

According to the present application, in step (1), the protonic acid catalyst can establish a stronger hydrogen bond network with water in the presence of the specific organic solvent used, so that the catalyst is soluble in the aqueous halogenated quaternary ammonium salt solution but insoluble in the organic solvent, therefore after the reaction is completed and after the organic solvent and the aqueous halogenated quaternary ammonium salt solution are separated into phases, the acid catalyst is substantially retained in the aqueous halogenated quaternary ammonium salt solution in the lower layer, and is separated in situ from the target product in the upper layer. At this time, the aqueous phase solution is a mixed aqueous solution of the halogenated quaternary ammonium salt and the liquid acid, and it can be directly recovered by the liquid separation without the necessity of further separating the halogenated quaternary ammonium salt from the acid catalyst. It can be directly reused after decolourization. A small amount of the acid catalyst remaining in the organic phase can be neutralized by adding an equal amount of an alkali. The generated salt will precipitate from the organic phase and can be directly filtered without affecting the solvent recovery. Thus, the difficult problem of separating the liquid acid catalyst from the target product can be solved, and the continuous production of 5-hydroxymethylfurfural can be achieved.

In a preferable embodiment, the step (1) further includes the phase separation of the dehydration reaction product to obtain an aqueous phase containing the acid catalyst and an organic phase containing 5-hydroxymethylfurfural. According to the present application, in step (1), during the dehydration of the fructose-based carbohydrate to prepare the target product, 5-hydroxymethylfurfural is extracted into the organic solvent in real time. Therefore, after the reaction is completed and after the organic solvent and the aqueous halogenated quaternary ammonium salt solution are separated into phases, 5-hydroxymethylfurfural is present in the organic solvent in the upper layer. At the same time, the by-products and impurities such as humins produced during the reaction remain in the organic phase, therefore the organic solvent in the upper layer appears brown. As a specific example, the phase separation process of the dual liquid phase reaction system of the dehydration reaction may include: after the reaction is completed and the stirring is terminated, due to the salting-out effect of the halogenated quaternary ammonium salt, the organic solvent and the aqueous halogenated quaternary ammonium salt solution will immediately be divided into upper- and lower-layer phases, wherein the upper-layer phase is the organic solvent and the lower-layer phase is the aqueous halogenated quaternary ammonium salt solution, and there is a clear phase interface between the two phases. The two phases can be separated by conventional processes, such as syringe extraction, separatory funnel separation, or other conventional processes in the art to separate and withdraw two phases.

In some further preferable embodiments, in step (1), after the phase separation, a part of the organic phase containing 5-hydroxymethylfurfural is decolorized, separated and purified to obtain 5-hydroxymethylfurfural. Preferably, the decolorization treatment can be performed by activated carbon adsorption, and the separation and purification process can include vacuum distillation of the decolorized 5-hydroxymethylfurfural solution, followed by crystallization or recrystallization, and then freeze-drying. Furthermore, the process of the present application can also include calcining the activated carbon that has adsorbed impurities at high temperature to restore the adsorption capacity of the activated carbon.

In a preferable embodiment, in step (1), the mass ratio of the fructose-based carbohydrate to the reaction medium is 1:1-1000, preferably 1:2-100, more preferably 1:5-20, wherein the reaction system is a dual liquid phase reaction medium composed of the polar organic solvent and the aqueous halogenated quaternary ammonium salt solution.

In a preferable embodiment, in step (1), the mass ratio of the fructose-based carbohydrate to the halogenated quaternary ammonium salt is 1:0.1-10, preferably 1:1-5.

In a preferable embodiment, in step (1), the proportion of the volume of the aqueous halogenated quaternary ammonium salt solution relative to the total volume of the reaction medium is 5-50%, preferably 10-35%.

In a preferable embodiment, in step (1), based on the mass of protonic acid, the concentration of the protonic acid catalyst in the aqueous phase of the reaction medium is 0.02-0.5 g/mL, preferably 0.03-0.1 g/mL.

In a preferable embodiment, in step (1), the conditions for the dehydration reaction include: reaction temperature of 80-200°C, preferably 100-150°C; reaction time of 0.1-12 hours, preferably 0.1-1 hour.

According to the present application, the reactor used for the dehydration reaction in step (1) can be a thick walled pressure-resistant vessel, a stainless steel reactor with a polytetrafluoroethylene (PTFE) lining, and other commonly used reactors in the art. In order to facilitate the observation of the phase interface between the aqueous and organic phases and further facilitate the separation operation of the two phases, the reactor is preferably a thick walled pressure-resistant vessel.

According to the present application, the organic phase containing 5-hydroxymethylfurfural obtained in step (1), due to a relatively high content of 5-hydroxymethylfurfural and a relatively low content of impurities therein, can be directly used as raw material for producing 2,5-furanedicarboxylic acid to perform the oxidation reaction described in step (2) in the presence of an oxidation catalyst and oxygen gas, without subjected to the process of separation and purification.

In a preferable embodiment, the oxidation reaction of step (2) is performed without the presence of alkali.

According to the present application, the oxidation catalyst used in step (2) is a loaded metal catalyst, preferably comprising a support and an active metal component loaded on the support. The active metal component comprises a precious metal selected from Ru, Pt, Pd, Au or combinations thereof, preferably Ru. In a preferable embodiment, the active metal component of the oxidation catalyst is Ru or a combination of Ru with one or more selected from Pt, Pd and Au. For example, the active metal component can be a combination of Ru with one or more selected from Pt, Pd and Au, and based on the total mass of the active metal components, the mass content of Ru is 5-100%, preferably 20-100%, more preferably 40-100%, further preferably 50-100%, particularly preferably 60-100%.

In a preferable embodiment, based on the total mass of the support, the content of the active metal component in the oxidation catalyst is 0.25-30%, preferably 1-20%, more preferably 5-15%, particularly preferably the content of Ru is 5-15%.

In a preferable embodiment, the support is a carbon-containing material, preferably selected from activated carbon, carbon nanotube, graphene, graphene oxide, or combinations thereof, more preferably activated carbon. In a further preferable embodiment, the support is an activated carbon having a specific surface area of 1000-2000 m²/g, preferably 1000-1500 m²/g; particularly preferably, the activated carbon has a pore size of about 1-5 nm. The inventors of the present application unexpectedly found that using a Ru-based catalyst loaded on a carbon-containing material (such as activated carbon, etc.) with a high specific surface area (1000-2000 m²/g) is more conducive to the reaction process of HMF oxidation to prepare FDCA in a mixed solvent composed of water and an organic solvent under an alkali-free condition. Moreover, the activated carbon with a high specific surface area (1000-2000 m²/g) can be used as a support without being treated with alkaline nitrogen-containing compounds, resulting in a Ru-based catalyst with excellent performance, thereby simplifying the catalyst preparation process and reducing the cost. Therefore, it is particularly preferred that the oxidation catalyst used in the present application is substantially free of nitrogen element, for example, the nitrogen element content is less than 0.01% by weight of the catalyst.

According to the present application, the oxidation catalyst can be prepared according to existing processes, such as isovolumetric impregnation process, incipient wetness impregnation process, ion exchange process, deposition-precipitation process or vacuum impregnation process, and the like. For example, in some specific embodiments, after depositing the active metal component on the support, the resulting solid powder can be dried in an oven at 100-140°C for about 6-24 hours, the obtained catalyst precursor is first calcined in nitrogen gas at 300-800°C for a period of time, and then reduced in a reducing atmosphere (such as H₂ or a mixed atmosphere of H₂ and N₂) at a temperature of 200-500°C for about 6-24 hours to obtain the oxidation catalyst. The preparation process of the oxidation catalyst used in the present application is simple, inexpensive, easy to scale up production, and has good industrial application prospects.

In a preferable embodiment, in step (2), the mass ratio of the organic phase containing 5-hydroxymethylfurfural to water is 5:1-0.5:1, preferably 3:1-1:1.

In a preferable embodiment, in step (2), the oxidation catalyst is used in such an amount that the molar ratio of the active metal component in the oxidation catalyst to the 5-hydroxymethyl furfural is 1:1-1000, preferably 1:5-500, more preferably 1:5-250.

In a preferable embodiment, in step (2), the conditions of the oxidation reaction comprise: reaction temperature of 50-170°C, preferably 90-150°C, oxygen gas partial pressure of 0.2-4 MPa, preferably 1-3 MPa, further preferably, the oxidation reaction is performed under an atmosphere of oxygen gas, an atmosphere of air, or a mixed atmosphere of oxygen gas and nitrogen gas.

In a preferable embodiment, step (2) further comprises separating and purifying the product of the 2,5-furandicarboxylic acid solution obtained from the oxidation reaction to obtain the 2,5-furandicarboxylic acid. More preferably, the separation and purification process comprises subjecting the solution containing 2,5-furandicarboxylic acid to vacuum distillation, with the distillation temperature of 30-100°C, preferably 30-50°C and the distillation time of 1-10 hours, preferably 2-4 hours; alternatively, the separation and purification process comprises subjecting the solution containing 2,5-furandicarboxylic acid to crystallization or recrystallization, with the crystallization temperature of -10 to 30°C, preferably 0 to 20°C and the crystallization time of 1-24 hours, preferably 5-10 hours. Still more preferably, the purification process further comprises a process of drying the product after distillation or crystallization, with the drying process preferably being freeze-drying. The drying time is 3-24 hours, preferably 18-24 hours.

In the second aspect, the present application provides a process for producing 2,5-furandicarboxylic acid, comprising the following steps:
in a mixed solvent containing an organic solvent and water, in the presence of an oxidation catalyst and oxygen gas, subjecting 5-hydroxymethylfurfural to an oxidation reaction to produce 2,5-furandicarboxylic acid;
wherein the oxidation catalyst comprises a support and an active metal component loaded on the support, wherein the support is an activated carbon having a specific surface area of 1000-2000 m²/g, preferably 1000-1500 m²/g, and the active metal component contains Ru.

In a preferable embodiment, the oxidation reaction of the process of the present application is performed without the presence of alkali.

In a preferable embodiment, the organic solvent used is an aprotic polar organic solvent miscible with water, preferably selected from acetone, tetrahydrofuran, 1,4-dioxane, acetonitrile, dimethyl sulfoxide, or combinations thereof, more preferably 1,4-dioxane;
In the oxidation reaction of the processes in the first and second aspects of the present application, on the one hand, a mixed solvent formed from the organic solvent in the raw material and the added water can increase the solubility of the product of 2,5-furandicarboxylic acid, thereby avoiding the introduction of alkaline compounds and also avoiding the generation of salt products. A solution containing 2,5-furandicarboxylic acid is directly obtained, and after separation and purification of the solution, 2,5-furandicarboxylic acid can be obtained, simplifying the product post-treatment step and avoiding the generation of a large amount of waste acid and wastewater in the subsequent acidification process. On the other hand, the price of the active metal Ru used in the oxidation catalyst is significantly lower than that of metals such as Pt or Au. At the same time, the catalyst can be prepared with a simple process from commercially available activated carbon and metal precursors, and the FDCA yield is high (>90%). Compared with the disclosed processes, the process of the present application greatly simplifies the process flow and reduces the cost. The reaction process is green and concise, and the FDCA yield is high. It is a process for preparing FDCA with great industrial potential.

In a preferable embodiment, the active metal component of the oxidation catalyst is Ru or a combination of Ru with one or more selected from Pt, Pd and Au. For example, the active metal component can be Ru or a combination of Ru with one or more selected from Pt, Pd and Au, and based on the total mass of the active metal component, the mass content of Ru is 40-100%, preferably 50-100%, more preferably 60-100%. According to the second aspect of the present application, other features of the oxidation catalyst may be those as described in the first aspect of the present application, and will not be described again here.

In a preferable embodiment, the mixed solvent consists of the organic solvent and water. Further preferably, in the mixed solvent, the mass ratio of the organic solvent to water is 5:1-0.5:1, preferably 3:1-1:1.

In a preferable embodiment, in a solution formed from the 5-hydroxymethylfurfural and the mixed solvent, the mass percentage of the 5-hydroxymethylfurfural is 0.1-30%, preferably 0.5-20%, more preferably 1-10%.

In a preferable embodiment, the oxidation catalyst is used in such an amount that the molar ratio of the active metal component in the oxidation catalyst to the 5-hydroxymethyl furfural is 1:1-1000, preferably 1:5-500, more preferably 1:5-250.

In a preferable embodiment, the conditions of the oxidation reaction comprise: reaction temperature of 50-170°C, preferably 90-150°C, oxygen gas partial pressure of 0.2-4 MPa, preferably 1-3 MPa, further preferably, the oxidation reaction is performed under an atmosphere of oxygen gas, an atmosphere of air, or a mixed atmosphere of oxygen gas and nitrogen gas.

In the third aspect, the present application provides a reaction system for continuously producing 5-hydroxymethylfurfural and 2,5-furandicarboxylic acid, which comprises a dehydration reaction unit, a phase separation unit, and an oxidation reaction unit, the dehydration reaction unit has an inlet and an outlet, the phase separation unit has an inlet, an aqueous phase outlet and an organic phase outlet, the oxidation reaction unit has an inlet and an outlet, the outlet of the dehydration reaction unit is communicated with the inlet of the phase separation unit, the aqueous phase outlet of the phase separation unit is communicated with the inlet of the dehydration reaction unit, and the organic phase outlet of the phase separation unit is communicated with the inlet of the oxidation reaction unit, wherein a fructose-based carbohydrate is subjected to a dehydration reaction in a dual liquid phase reaction medium containing a polar organic solvent and an aqueous halogenated quaternary ammonium salt solution in the presence of a protonic acid catalyst in the dehydration reaction unit to produce a two-phase stream containing 5-hydroxymethylfurfural, the two-phase stream is subjected to a phase separation in the phase separation unit to produce an aqueous phase containing the acid catalyst and an organic phase containing 5-hydroxymethylfurfural, the aqueous phase containing the acid catalyst is fed back to the dehydration reaction unit, and the organic phase containing 5-hydroxymethylfurfural is subjected to an oxidation reaction in the oxidation reaction unit to produce a stream containing 2,5-furandicarboxylic acid.

In a preferable embodiment, the dehydration reaction unit includes a dehydration reactor selected from a slurry bed, a bubbling bed, a shell and tube reactor or combinations thereof. In a preferable embodiment, the oxidation reaction unit includes an oxidation reactor selected from a slurry bed, a fixed bed, a bubbling bed, or combinations thereof.

In a preferable embodiment, the phase separation unit comprises a phase separation tank and the like.

In a preferable embodiment, the reaction system further comprises a purification unit and a drying unit, and the stream containing 2,5-furandicarboxylic acid from the oxidation reaction unit is treated in the purification unit and the drying unit to obtain a product of 2,5-furandicarboxylic acid.

In a further preferable embodiment, the purification unit can be a distillation separation unit, wherein the separation equipment can be a vacuum distillation column or a molecular distiller; it can also be a crystallization separation unit, where the separation equipment can be a fluidized bed, a tubular continuous crystallizer or a precipitation crystallizer, and the like; it can also be an adsorption separation unit, where the separation equipment can be an activated carbon adsorption column.

In a further preferable embodiment, the drying unit includes a dryer selected from a spray dryer, a fluidized bed dryer, a pneumatic dryer, a box-type dryer or combinations thereof. In a preferable embodiment, the reaction system further comprises a decolorization and purification unit and a freeze-drying unit, wherein a part of the organic phase containing 5-hydroxymethylfurfural from the phase separation unit is treated in the decolorization and purification unit and the freeze-drying unit to obtain a product of 5-hydroxymethylfurfural. Particularly preferably, the decolorization and purification unit comprises an activated carbon adsorption column, a vacuum distillation column and a crystallizer; and the freeze-drying unit comprises a freeze-dryer.

In a preferable embodiment, the reaction system of the present application may also include an activated carbon calcining furnace and a corresponding exhaust gas absorption device.

A preferable embodiment of the reaction system for continuously producing 5-hydroxymethylfurfural and 2,5-furandicarboxylic acid of the present application will be described in detail below in conjunction with the accompanying drawing.

As shown in Figure 1, the reaction system comprises a dehydration reaction unit, a phase separation unit, an oxidation reaction unit, a purification unit and a drying unit, wherein the fructose-based carbohydrate raw material is subjected to an intramolecular dehydration reaction in a dual liquid phase reaction medium containing a polar organic solvent and an aqueous halogenated quaternary ammonium salt solution in the presence of a protonic acid catalyst in the dehydration reaction unit to produce a two-phase stream containing 5-hydroxymethylfurfural. The two-phase stream is subjected to a phase separation in the phase separation unit to produce an aqueous phase containing the acid catalyst and an organic phase containing 5-hydroxymethylfurfural. The aqueous phase containing the acid catalyst is fed back to the dehydration reaction unit. The organic phase containing 5-hydroxymethylfurfural is subjected to an oxidation reaction in the oxidation reaction unit to produce a solution containing 2,5-furandicarboxylic acid. The solution containing 2,5-furandicarboxylic acid is subjected to separation and/or purification in the purification unit, and then dried in the drying unit to obtain the product of 2,5-furandicarboxylic acid.

In a preferable embodiment, the present application provides the following technical solutions:
1. A process for continuously producing 5-hydroxymethylfurfural and 2,5-furandicarboxylic acid, comprising:
   (1) in a dual phase reaction system composed of a low boiling point polar organic solvent and an aqueous halogenated quaternary ammonium salt solution, under the catalysis of a strong acidic liquid acid catalyst containing sulfonic acid groups, subjecting a fructose-based carbohydrate to an intramolecular dehydration reaction in a reactor; after the reaction is completed, the reaction system is divided into an aqueous phase and an organic phase containing 5-hydroxymethylfurfural; and
   (2) using the organic phase containing 5-hydroxymethylfurfural in step (1) as raw material, adding water, and subjecting the 5-hydroxymethylfurfural to an oxidation reaction in a reactor in the presence of an oxidation catalyst and oxygen gas to produce a solution containing 2,5-furandicarboxylic acid, which is subjected to separation and purification to obtain the 2,5-furandicarboxylic acid;
   wherein the oxidation catalyst is a loaded metal catalyst.
2. The process according to item 1, wherein, in step (1), the fructose-based carbohydrate is one of purified fructose, crude fructose, polyfructose, fructose syrup, and fructose dextrose syrup.
3. The process according to item 1, wherein, in step (1), the liquid acid catalyst is selected from sulfuric acid, methylsulfonic acid or combinations thereof.
4. The process according to item 1, wherein, in step (1), the halogenated quaternary ammonium salt is selected from C₁-C₁₂ hydrocarbyl and hydroxy or halogen-substituted C₁-C₁₂ hydrocarbyl halogenated quaternary ammonium salts, preferably one or more of C₁-C₆ hydrocarbyl and hydroxy or halogen-substituted C₁-C₆ hydrocarbyl trimethylammonium halides, further preferably one or more of C₁-C₄ hydrocarbyl and hydroxy or halogen-substituted C₁-C₄ hydrocarbyl trimethylammonium chloride or bromide.
5. The process according to item 1, wherein, in step (1), the halogenated quaternary ammonium salt is one of tetramethylammonium chloride, choline chloride, and chlorocholine chloride.
6. The process according to item 1, wherein, in step (1), the polar organic solvent is one or two of acetone, tetrahydrofuran, 1,4-dioxane, and acetonitrile, preferably 1,4-dioxane.
7. The process according to item 1, wherein, in step (1), the mass ratio of the fructose-based carbohydrate to the reaction system is 1:1-1000, preferably 1:2-100, more preferably 1:5-20, wherein the reaction system refers to the total of the polar organic solvent and the aqueous halogenated quaternary ammonium salt solution.
8. The process according to item 1, wherein, in step (1), the aqueous halogenated quaternary ammonium salt solution comprises 5-50%, preferably 10-35% by volume of the total reaction system.
9. The process according to item 1, wherein, in step (1), the concentration of the liquid acid catalyst in the aqueous phase is 0.02-0.5 g/mL, preferably 0.03-0.1 g/mL.
10. The process according to item 1, wherein, in step (1), the temperature of the dehydration reaction is 80-200°C, preferably 100-150°C; the time of the dehydration reaction is 0.1-12 hours, preferably 0.1-1 hour.
11. The process according to item 1, wherein, in step (2), the loaded metal catalyst comprises a support and a metal loaded on the support, the metal is Ru, or a combination composed of Ru with one or more of Pt, Pd or Au.
12. The process according to item 11, wherein, in step (2), in the combination composed of Ru with one or more of Pt, Pd or Au, the mass content of Ru is 5-95%, preferably 20-90%, more preferably 40-80%.
13. The process according to item 11, wherein, in step (2), in the loaded metal catalyst, the metal is loaded in an amount of 0.25-30%, preferably 1-20%, more preferably 5-15%, based on the total mass of the support.
14. The process according to item 11, wherein, in step (2), the support is selected from carbon-containing materials, comprising one or more of activated carbon, carbon nanotube, graphene and graphene oxide, preferably activated carbon.
15. The process according to item 14, wherein in case that the support in step (2) is activated carbon, the activated carbon has a specific surface area of 1000-1500 m²/g.
16. The process according to item 1, wherein, in step (2), the mass ratio of the organic phase containing 5-hydroxymethylfurfural to water is 5:1-0.5:1, preferably 3:1-1:1.
17. The process according to item 1, wherein, in step (2), the molar ratio of the metal in the loaded metal catalyst to the 5-hydroxymethylfurfural is 1:1-1000, preferably 1:5-500, more preferably 1:5-250.
18. The process according to item 1, wherein, in step (2), the oxygen gas is derived from pure oxygen, air or a mixed gas composed of oxygen gas and nitrogen gas, the oxygen gas has a partial pressure of 0.2-4 MPa, preferably 1-3 MPa.
19. The process according to item 1, wherein, in step (2), the reaction temperature of the oxidation reaction is 50-170°C, preferably 90-150°C.
20. A reaction system for continuously producing 5-hydroxymethylfurfural and 2,5-furandicarboxylic acid, which comprises: a dehydration reaction unit, a phase separation unit, an oxidation reaction unit, a separation and purification unit, and a drying unit.
21. The reaction system according to item 20, wherein in the dehydration reaction unit, the fructose-based carbohydrates are dehydrated to prepare 5-hydroxymethylfurfural, wherein the dehydration reactor is a slurry bed, a bubbling bed or a shell and tube reactor, and after the reaction is completed, the dual liquid phase reaction system is fed to the phase separation unit to automatically separate into phases.
22. The reaction system according to item 20, wherein the organic phase containing 5-hydroxymethylfurfural that is separated from the phase separation unit is fed to the reactor of the oxidation reaction unit, and the oxidation reactor is a slurry bed, a fixed bed, or a bubbling bed.
23. The reaction system according to item 20, wherein the reaction solution containing 2,5-furandicarboxylic acid obtained from the oxidation reaction unit is passed through the separation and purification unit and the drying unit to obtain the product of 2,5-furandicarboxylic acid.
24. The reaction system according to item 20, wherein the reactor of the separation and purification unit is selected from a vacuum distillation column, a molecular distiller, a fluidized bed, a tubular continuous crystallizer or a precipitation crystallizer.
25. The reaction system according to item 20, wherein the organic phase containing 5-hydroxymethylfurfural that is separated from the phase separation unit is partially fed to the decolorization and purification unit and the freeze-drying unit to obtain the product of 2,5-furandicarboxylic acid.
26. The reaction system according to item 25, wherein the decolorization and purification unit includes an activated carbon adsorption column, a vacuum distillation column, and a crystallizer, and the freeze-drying unit includes a freeze-dryer.

### Examples

Unless otherwise specified, the experimental methods used in the following examples and comparative examples are conventional methods.

The materials, reagents, and the like used in the following examples and comparative examples can be obtained from commercial sources unless otherwise specified, and their purity is of analytical purity grade, wherein the source of 5-hydroxymethylfurfural was Beijing Innochem Science and Technology Co. Ltd.; the source of activated carbon was Beijing Dali Hongye Technology Co., Ltd. (analytically pure, hereinafter referred to as "Dali Hongye"), Calgon Carbon (Suzhou) Co., Ltd. (brand: OVC 4x8, 107C), Cabot (China) Investment Co., Ltd. (brand: VXC72); methylsulfonic acid and sulfamic acid were purchased from China Sinopharm Chemical Reagent Co., Ltd.; tetramethyl ammonium chloride was purchased from Beijing Innochem Science and Technology Co. Ltd.

In the following examples and comparative examples, high performance liquid chromatography was used to analyze and calculate the conversion rate of the reaction substrate, and the selectivity, yield and purity of the target product during the reaction process.

### Preparation Examples 1-6

The oxidation catalyst 10%Ru/C was prepared using the incipient wetness impregnation method:
An aqueous RuCl₃ solution containing 0.1 g Ru and 10.0 mL deionized water were mixed and stirred evenly. Then 1.0 g of activated carbon support was added to the mixed solution. The impregnation was performed under stirring at room temperature for 10 hours. The moisture was evaporated to dryness. Then the resulting solid was placed in an oven at 110°C and dried for 12 hours to produce a catalyst precursor. The loading amount of Ru was 10% (by mass). The precursor prepared in the above steps was placed in a quartz tube, first calcined in nitrogen at 500°C for 4 hours, and then reduced in 20% H₂+N₂ at 500°C for 3 hours to produce a loaded 10%Ru/C catalyst C1.

Different catalysts C2-C6 with various loading amounts of Ru on various commercially available activated carbon powders or particles were prepared in a similar manner. The compositions and specific surface areas of these catalysts are listed in Table I-1 below.

### Preparation Examples 7-11

The oxidation catalyst 10%Ru-5%Pt/C was prepared using the incipient wetness impregnation method:
An aqueous RuCl₃ solution containing 0.1 g Ru, an H₂PtCl₆ solution containing 0.05 g Pt and 10.0 mL deionized water were mixed and stirred evenly. Then 1.0 g of activated carbon support was added to the mixed solution. The impregnation was performed under stirring at room temperature for 10 hours. The moisture was evaporated to dryness. Then the resulting solid was placed in an oven at 110°C and dried for 12 hours to produce a catalyst precursor. The loading amount of Ru was 10% (by mass), and the loading amount of Pt was 5% (by mass). The precursor prepared in the above steps was placed in a quartz tube, first calcined in nitrogen at 500°C for 4 hours, and then reduced in 20% H₂+N₂ at 500°C for 3 hours to produce a loaded 10%Ru-5%Pt/C catalyst C7.

Different catalysts C8-C11 with various loading amounts of Ru and Pt/Pd/Au on various commercially available activated carbon powders or particles were prepared in a similar manner. The compositions and specific surface areas of these catalysts are listed in Table I-1 below.

### Preparation Examples 8-12

Following the procedure of Preparation Example 1, different catalysts D1-D5 with various loading amounts of Pt or Au on various commercially available activated carbon powders or particles were prepared. The compositions and specific surface areas of these catalysts are listed in Table I-3 below.

### Preparation Examples 13-14

Following the procedure of Preparation Example 1, different catalysts D6-D7 with various loading amounts of Ru on commercially available silica (SiO₂) or zirconia (ZrO₂) powders or particles were prepared. The compositions and specific surface areas of these catalysts are listed in Table I-4 below.

### Preparation Examples 15-16

According to the method reported in the preparation examples 1-2 of patent CN111039906A, different catalysts D8-D9 with various loading amounts of Ru on activated carbon powder or particles treated with alkaline nitrogen-containing compounds were prepared. The compositions and specific surface areas of these catalysts are listed in Table I-5 below.

### Examples I-1 to I-4

This example is used to illustrate the process of synthesizing 2,5-furandicarboxylic acid according to the present application.

To a 50 mL high-pressure reactor were added 0.5 g of 5-hydroxymethylfurfural, 0.2 g of 10% Ru/C oxidation catalysts C1-C4 obtained in Preparation Examples 1-4, 10 g of the mixed solvent composed of water and 1,4-dioxane (the mass ratio of water to 1,4-dioxane = 1:1). After the reactor was sealed, 1 MPa of oxygen was filled to replace the residual air in the reactor. After three repetitions, 1 MPa of oxygen was filled to the reactor, and the reactor was placed on a heating furnace for heating to a reaction temperature of 100°C. The reaction was performed under stirring at a rotation rate of 700 rpm for 10 hours. After the reaction was completed, the reactor was taken out of the heating furnace and cooled to room temperature. Filtration was conducted, washing the filter cake with the same mixed solvent as the reaction solvent and finally setting the volume to 100 mL. A liquid sample was taken for high-performance liquid chromatography analysis. The reaction results are listed in Table I-1.

### Examples I-5 to I-6

The experiments were carried out according to the method of Example I-1, except that the Ru/C catalysts C5-C6 loaded on the Dali Hongye activated carbon obtained in Preparation Examples 5-6 were used as the oxidation catalyst, and the loading amount of Ru in the oxidation catalyst was 5% or 15%. The reaction results are listed in Table I-1.

### Examples I-7 to I-8

The experiments were carried out according to the method of Example I-1, except that the 10%Ru/C catalyst C1 loaded on the Dali Hongye activated carbon obtained in Preparation Example 1 was used as the oxidation catalyst, and the oxygen pressure was 2 MPa or 3 MPa. The reaction results are listed in Table I-1.

### Examples I-9 to I-10

The experiments were carried out according to the method of Example I-1, except that the 10%Ru/C catalyst C1 loaded on the Dali Hongye activated carbon obtained in Preparation Example 1 was used as the oxidation catalyst, and the reaction temperature was 120°C or 140°C. The reaction results are listed in Table I-1.

### Examples 1-11 to 1-15

The experiments were carried out according to the method of Example I-1, except that the catalysts C7-C11 loaded on the Dali Hongye activated carbon obtained in Preparation Examples 7-11 were used as the oxidation catalyst. The reaction results are listed in Table 1-1.

**Table I-1. Catalyst compositions used in Examples I-1 to 1-15 and reaction results**

| Example No. | Catalyst | Carbon support source | Carbon support Specific surface area /m²/g | Metal loading amount | Oxygen gas pressure /MPa | Reaction temperature /°C | HMF conversion rate /% | FDCA yield /% |
|---|---|---|---|---|---|---|---|---|
| I-1 | C1 | Dali Hongye | 1286 | 10% Ru | 1 | 100 | 100 | 95 |
| I-2 | C2 | Calgon OVC 4x8 | 1013 | 10% Ru | 1 | 100 | 100 | 91 |
| I-3 | C3 | Calgon 107C | 804 | 10% Ru | 1 | 100 | 93 | 77 |
| I-4 | C4 | Cabot VXC72 | 258 | 10% Ru | 1 | 100 | 45 | 23 |
| I-5 | C5 | Dali Hongye | 1286 | 5% Ru | 1 | 100 | 100 | 92 |
| I-6 | C6 | Dali Hongye | 1286 | 15% Ru | 1 | 100 | 100 | 91 |
| I-7 | C1 | Dali Hongye | 1286 | 10% Ru | 2 | 100 | 100 | 92 |
| I-8 | C1 | Dali Hongye | 1286 | 10% Ru | 3 | 100 | 100 | 90 |
| I-9 | C1 | Dali Hongye | 1286 | 10% Ru | 1 | 120 | 100 | 90 |
| I-10 | C1 | Dali Hongye | 1286 | 10% Ru | 1 | 140 | 100 | 86 |
| I-11 | C7 | Dali Hongye | 1286 | 10% Ru -5% Pt | 1 | 100 | 100 | 93 |
| I-12 | C8 | Dali Hongye | 1286 | 8% Ru- 7% Pt | 1 | 100 | 100 | 91 |
| I-13 | C9 | Dali Hongye | 1286 | 12% Ru- 3% Pt | 1 | 100 | 100 | 92 |
| I-14 | C10 | Dali Hongye | 1286 | 10% Ru- 5% Pd | 1 | 100 | 100 | 87 |
| I-15 | C11 | Dali Hongye | 1286 | 10% Ru- 5% Au | 1 | 100 | 100 | 90 |

It can be seen from the data in Table I-1:
1. The specific surface area of the activated carbon support in the Ru/C oxidation catalyst had a significant effect on the activity of the catalyst and the yield of FDCA. When the specific surface area of the activated carbon support was greater than 1000 m²/g, the yield of FDCA reached 90% or higher;
2. The loading amount of Ru in the Ru/C oxidation catalyst would affect the performance of the catalyst, but within the loading amount range of 5% to 15%, the FDCA yield was greater than 90%;
3. The oxygen gas partial pressure and the reaction temperature would also affect the performance of the Ru/C oxidation catalyst. Too high oxygen gas partial pressure and reaction temperature would cause a decrease in the FDCA yield; and
4. The oxidation catalyst composed of Ru and Pt/Pd/Au loaded on activated carbon also had relatively good catalytic performance under the investigation conditions of the present application.

### Examples I-16 to I-17

The experiments were carried out according to the method of Example I-1, except that the 10%Ru/C catalyst C1 loaded on the Dali Hongye activated carbon obtained in Preparation Example 1 was used as the oxidation catalyst, and the reaction solvent was a mixed solvent composed of water and tetrahydrofuran (the mass ratio of water to tetrahydrofuran = 1:1), or a mixed solvent composed of water and dimethyl sulfoxide (the mass ratio of water to dimethyl sulfoxide = 1:1). The reaction results are listed in Table I-2.

### Examples I-18 to I-19

The experiments were carried out according to the method of Example I-1, except that the 10%Ru/C catalyst C1 loaded on the Dali Hongye activated carbon obtained in Preparation Example 1 was used as the oxidation catalyst, and the reaction solvent was a mixed solvent composed of water and 1,4-dioxane (the mass ratio of water to 1,4-dioxane = 1:2 or 1:3). The reaction results are listed in Table I-2.

### Examples I-20 to I-21

The experiments were carried out according to the method of Example I-1, except that the 10%Ru/C catalyst C1 loaded on the Dali Hongye activated carbon obtained in Preparation Example 1 was used as the oxidation catalyst, and the total mass of the reaction solvent was 5 g or 20 g. The reaction results are listed in Table I-2.

**Table I-2.The influence of the solvent compositions and the total solvent addition amounts in Examples I-1 and I-16 to I-21 on the reaction results.**

| Example | Solvent (mass ratio) | Total solvent addition amount /g | Oxygen gas pressure /MPa | Reaction temperature /°C | HMF conversion rate /% | FDCA yield /% |
|---|---|---|---|---|---|---|
| I-1 | water: 1,4-dioxane = 1:1 | 10 | 1 | 100 | 100 | 95 |
| I-16 | water:tetrahydrofuran = 1:1 | 10 | 1 | 100 | 100 | 92 |
| I-17 | water: dimethyl sulfoxide = 1:1 | 10 | 1 | 100 | 100 | 85 |
| I-18 | water: 1,4-dioxane=1 :2 | 10 | 1 | 100 | 100 | 94 |
| I-19 | water: 1,4-dioxane=1:3 | 10 | 1 | 100 | 100 | 93 |
| I-20 | water: 1,4-dioxane = 1: 1 | 5 | 1 | 100 | 100 | 87 |
| I-21 | water: 1,4-dioxane = 1: 1 | 20 | 1 | 100 | 100 | 97 |

It can be seen from the data in Table I-2 that solvents composed of water and 1,4-dioxane, tetrahydrofuran or dimethyl sulfoxide at different mass ratios within the limited range of the present application could effectively achieve the reaction process of HMF oxidation to prepare FDCA. The total solvent addition amount would affect the performance of the catalyst. An increase in the total solvent addition amount, that is, a decrease in the concentration of 5-hydroxymethylfurfural, would contribute to increase the yield of FDCA, but it would also correspondingly reduce the one-way processing capacity of this process.

### Examples I-22 to I-26

The experiments were carried out according to the method of Example I-1, except that the catalysts D1-D5 prepared in Preparation Examples 8-12 in which Pt, Pd or Au was loaded on activated carbon were used as the oxidation catalyst. The reaction results are listed in Table I-3.

**Table I-3. Composition and performance of catalysts used in Examples I-22 to I-26**

| Example No. | Catalyst | Carbon support | Specific surface area /m²/g | Metal loading amount | Oxygen gas pressure /MPa | Reaction temperature /°C | HMF conversion rate /% | FDCA yield /% |
|---|---|---|---|---|---|---|---|---|
| I-22 | D1 | Dali Hongye | 1286 | 5% Pt | 1 | 100 | 40 | 27 |
| I-23 | D2 | Dali Hongye | 1286 | 10% Pt | 1 | 100 | 61 | 45 |
| I-24 | D3 | Dali Hongye | 1286 | 15% Pt | 1 | 100 | 87 | 63 |
| I-25 | D4 | Dali Hongye | 1286 | 10% Pd | 1 | 100 | 32 | 20 |
| I-26 | D5 | Dali Hongye | 1286 | 10% Au | 1 | 100 | 48 | 30 |

It can be seen from the data in Table I-3 that all of the oxidation catalysts in which Pt, Pd, or Au was loaded on the activated carbon alone within the limited range of the present application exhibited significantly lower catalytic performance than the Ru/C oxidation catalyst, indicating that the Ru/C oxidation catalyst loaded on the carbon-containing material having a high specific surface area involved in the present application had a special catalytic effect on the reaction of oxidizing HMF to FDCA.

### Examples I-27 to I-28

The experiments were carried out according to the method of Example I-1, except that the catalysts D6-D7 prepared in Preparation Examples 13-14 in which Ru was loaded on SiO₂ or ZrO₂ were used as the oxidation catalyst. The reaction results are listed in Table I-4.

**Table I-4. Composition and performance of catalysts used in Examples I-27 to I-28**

| Example No. | Catalyst | Support | Specific surface area /m²/g | Metal loading amount | Oxygen gas pressure /MPa | Reaction temperature /°C | HMF conversion rate /% | FDCA yield /% |
|---|---|---|---|---|---|---|---|---|
| I-27 | D6 | SiO₂ | 274 | 10% Ru | 1 | 100 | 51 | 33 |
| I-28 | D7 | ZrO₂ | 68 | 5% Ru | 1 | 100 | 23 | 14 |

It can be seen from the data in Table I-4 that both of the oxidation catalysts in which Ru was loaded on SiO₂ or ZrO₂ within the limited range of the present application exhibited significantly lower catalytic performance than the Ru/C oxidation catalyst used in the present application.

### Examples I-29 to I-30

The experiments were carried out according to the method of Example I-1, except that the catalysts D8-D9 prepared in Preparation Examples 15-16 in which Ru was loaded on activated carbon treated with alkaline nitrogen-containing compound were used as the oxidation catalyst. The reaction results are listed in Table I-5.

**Table I-5. Composition and performance of catalysts used in Examples I-29 to I-30**

| Example No. | Catalyst | Carbon support | Specific surface area /m²/g | Metal loading amount | Oxygen gas pressure /MPa | Reaction temperature /°C | HMF conversion rate /% | FDCA yield /% |
|---|---|---|---|---|---|---|---|---|
| I-29 | D8 | treated with melamine | 217 | 4.7% Ru | 1 | 100 | 100 | 91 |
| 1-30 | D9 | treated with biimidazole | 224 | 5.6% Ru | 1 | 100 | 100 | 89 |

Comparing the data in Tables I-1 and I-5, it can be seen that the catalytic performance of the Ru/C oxidation catalyst loaded on the activated carbon with high specific surface area used in the present application is comparable to that of the catalyst in which Ru was loaded on the activated carbon treated with alkaline nitrogen-containing compound reported in patent CN111039906A. However, the catalyst used in the present application did not require the treatment with alkaline nitrogen-containing compound, making its preparation more convenient and conducive to large-scale application.

### Comparative Examples I-1 to I-4

The experiments were carried out according to the method of Example I-1, except that the 10%Ru/C catalyst C1 loaded on the Dali Hongye activated carbon obtained in Preparation Example 1 was used as the oxidation catalyst, and the reaction solvent was pure water, pure 1,4-dioxane, pure tetrahydrofuran, or pure dimethyl sulfoxide. The reaction results are listed in Table I-4.

**Table I-4. The influence of the solvent compositions in Comparative Examples I-1 to I-4 on the reaction results.**

| Comparative Example No. | Solvent | Total solvent addition amount /g | Oxygen gas pressure /MPa | Reaction temperature /°C | HMF conversion rate /% | FDCA yield /% |
|---|---|---|---|---|---|---|
| I-1 | water | 10 | 1 | 100 | 11 | 1 |
| I-2 | 1,4-dioxane | 10 | 1 | 100 | 14 | 3 |
| I-3 | tetrahydrofuran | 10 | 1 | 100 | 10 | 1 |
| I-4 | dimethyl sulfoxide | 10 | 1 | 100 | 20 | 7 |

From the data in Table I-4, it can be seen that pure water, or 1,4-dioxane, tetrahydrofuran, or dimethyl sulfoxide solvents alone could not effectively dissolve the FDCA generated in the reaction, causing a small amount of FDCA generated in the reaction to adhere to the surface of the oxidation catalyst and poison the catalytic active site, resulting in a significant decrease in the activity of the oxidation catalyst and the yield of FDCA.

### Example II-1

This example illustrates the dehydration reaction step of synthesizing HMF in the present application using a sulfuric acid catalyst.

To a 15 mL pressure-resistant vessel were added 0.5 g of fructose, 0.06 g of sulfuric acid, 1.5 g of water, 8.5 g of 1,4-dioxane, and 0.5 g of tetramethylammonium chloride. After a multi-channel heater was heated to the temperature of 110°C, the pressure-resistant vessel was placed in the multi-channel heater and stirred for 25 minutes at a rotation rate of 600 r/min. After the reaction was completed, the pressure-resistant vessel was taken out from the multi-channel heater and cooled naturally to room temperature. The reaction solution was separated into an aqueous phase and an organic phase containing 5-hydroxymethylfurfural through a phase separator. The two phases were analyzed using high-performance liquid chromatography to determine the conversion rate of fructose and the yield of HMF. The yield of HMF and the corresponding conversion rate of fructose, as well as the entrapping rate of HMF in the upper-layer organic phase (i.e. the percentage of the HMF amount in the organic phase relative to the total HMF amount) are listed in Table II-1.

After the reaction was completed, the reaction solution was quickly divided into two phases, and the lower-layer organic quaternary ammonium salt aqueous solution was removed using a phase separator. The organic quaternary ammonium salt aqueous solution contained the acid catalyst. The content of the acid catalyst in the aqueous phase was determined by the alkaline titration method, and the recovery rate of the acid catalyst in the aqueous phase was further calculated based on the catalyst feed amount (i.e. the percentage of the amount of the acid catalyst in the aqueous phase relative to the acid catalyst feed amount). The results are listed in Table II-1.

### Example II-2

This example illustrates the dehydration reaction step of synthesizing HMF in the present application using a sulfuric acid catalyst.

The reaction was performed according to Example II-1, except that the feed amount of tetramethylammonium chloride was increased to 1.5 g. The corresponding reaction results are listed in Table II-1.

### Example II-3

This example illustrates the dehydration reaction step of synthesizing HMF in the present application using a sulfuric acid catalyst.

The reaction was performed according to Example II-1, except that the feed amount of tetramethylammonium chloride was increased to 2.5 g. The corresponding reaction results are listed in Table II-1.

After the reaction was completed, the reaction solution was quickly divided into two phases, and the lower-layer aqueous phase solution was removed using a phase separator and directly put into the next cycle test. The results of the cycle test are listed in Table II-2.

### Example II-4

This example illustrates the dehydration reaction step of synthesizing HMF in the present application using a methylsulfonic acid catalyst.

The reaction was performed according to Example II-1, except that the sulfuric acid catalyst was replaced with the methylsulfonic acid catalyst, and the feed amount of the catalyst was increased to 0.2 g. The corresponding reaction results are listed in Table II-1.

### Example II-5

This example illustrates the dehydration reaction step of synthesizing HMF in the present application using a methylsulfonic acid catalyst.

The reaction was performed according to Example II-2, except that the sulfuric acid catalyst was replaced with the methylsulfonic acid catalyst, and the feed amount of the catalyst was increased to 0.2 g. The corresponding reaction results are listed in Table II-1.

### Example II-6

This example illustrates the dehydration reaction step of synthesizing HMF in the present application using a methylsulfonic acid catalyst.

The reaction was performed according to Example II-3, except that the sulfuric acid catalyst was replaced with the methylsulfonic acid catalyst, and the feed amount of the catalyst was increased to 0.2 g. The corresponding reaction results are listed in Table II-1.

### Example II-7

The reaction was performed according to Example II-1, except that the feed amount of tetramethylammonium chloride was increased to 3.0 g. The corresponding reaction results are listed in Table II-1.

### Example II-8

The reaction was performed according to Example II-6, except that the feed amount of tetramethyl ammonium chloride was increased to 3.0 g. The corresponding reaction results are listed in Table II-1.

### Example II-9

This example illustrates the dehydration reaction step of synthesizing HMF in the present application using a sulfamic acid catalyst.

The reaction was performed according to Example II-1, except that the sulfuric acid catalyst was replaced with an equimolar amount of the sulfamic acid catalyst. The corresponding reaction results are listed in Table II-1.

### Comparative Example II-1

The reaction was performed according to Example II-1, except that tetramethyl ammonium chloride was replaced with an equimolar ratio of sodium chloride. The corresponding reaction results are listed in Table II-1.

### Comparative Example II-2

The reaction was performed according to Example II-2, except that tetramethyl ammonium chloride was replaced with an equimolar ratio of sodium chloride. The corresponding reaction results are listed in Table II-1.

### Comparative Example II-3

The reaction was performed according to Example II-3, except that tetramethyl ammonium chloride was replaced with an equimolar ratio of sodium chloride. The corresponding reaction results are listed in Table II-1.

**Table II-1: Experimental results of Examples II-1 to II-8 and Comparative Examples II-1 to II-3**

| Example | Fructose conversion rate (%) | HMF yield (%) | HMF entrapping rate (%) | Catalyst recovery rate (%) |
|---|---|---|---|---|
| Example II-1 | 99.5 | 84.0 | >95 | >94 |
| Example II-2 | 99.6 | 86.3 | >95 | >94 |
| Example II-3 | 99.5 | 88.0 | >95 | >94 |
| Example II-4 | 99.6 | 85.5 | >95 | >92 |
| Example II-5 | 99.4 | 88.0 | >95 | >92 |
| Example II-6 | 99.5 | 89.3 | >95 | >92 |
| Example II-7 | 99.4 | 88.1 | >95 | >94 |
| Example II-8 | 99.5 | 89.4 | >95 | >92 |
| Example II-9 | 99.5 | 83.7 | >95 | >95 |
| Comparative Example II-1 | 98.7 | 77.5 | >95 | >94 |
| Comparative Example II-2 | 98.7 | 77.8 | >95 | >94 |
| Comparative Example II-3 | 89.9 | 77.6 | >95 | >94 |

**Table II-2: Results of recycling the aqueous phase solvent in the dual liquid phase system in Example II-3**

| Cycle number | Fructose conversion rate (%) | HMF yield (%) |
|---|---|---|
| 1 | 99.5 | 88.0 |
| 2 | 99.5 | 88.1 |
| 3 | 99.4 | 87.9 |
| 4 | 99.1 | 87.8 |
| 5 | 99.0 | 87.2 |
| 6 | 99.0 | 86.8 |

Comparing the results of Examples II-1 to II-3 and Example II-7, it can be seen that after adding tetramethylammonium chloride to the reaction system, the HMF yield gradually increased as the feed amount of tetramethylammonium chloride increased, when the feed amount thereof increased from 2.5 g to 3.0 g, the HMF yield remained basically unchanged. Taking the HMF yield and the production cost into account together, the preferred mass ratio of fructose to tetramethylammonium chloride is 1:1-5.

Comparing the results of Examples II-4 to II-6 and Example II-8, it can be seen that after the catalyst was replaced with methylsulfonic acid, the trend of HMF yield changing with the feed amount of tetramethylammonium chloride, and the preferred mass ratio of fructose to tetramethylammonium chloride remained unchanged.

Comparing the results of Examples II-1, II-4, and II-9, it can be seen that under the same reaction conditions, when using sulfamic acid as catalyst, the yield of HMF was lower than that under the situation when using sulfuric acid or methylsulfonic acid as catalyst. This is because the use of sulfamic acid leads to the production of more by-product, humins. Therefore, compared to sulfamic acid, sulfuric acid and methylsulfonic acid are more preferred catalysts.

From the results in Table II-2, it can be seen that in Example II-3, after the reaction was completed, the lower-layer aqueous phase solvent of the dual liquid phase system was removed and directly put into the next cycle test. After 6 consecutive cycles, the fructose conversion rate remained above 99.0% without a significant decrease. The yield of HMF slightly decreased after 4 cycles, decreased to 87.2% in the 5th cycle reaction, and to 86.8% in the 6th cycle reaction, indicating that the aqueous phase solution after the phase separation had excellent recovery performance.

Comparing the results of Comparative Examples II-1 to II-3, it can be seen that when the salt compound used in the reaction system become inorganic salt sodium chloride, although a dual liquid phase system could still be formed, the yield of HMF no longer increased as the feed amount of sodium chloride increased. This indicated that unlike organic quaternary ammonium chloride salts, ordinary inorganic chloride salts had no promoting effect on the generation of HMF. And at the same salt concentration, the HMF yield for the organic quaternary ammonium salt was higher than that for inorganic chloride salts, indicating that the cation of the organic quaternary ammonium salt would promote the generation of HMF compared to the metal cation.

### Examples III-1 to III-4

This example is used to illustrate the process of synthesizing 2,5-furandicarboxylic acid using the product obtained in Example II-1 as raw material.

To a 50 mL high-pressure reactor were added 5 g of the organic phase containing 5-hydroxymethylfurfural obtained in Example II-1, 0.2 g of 10% Ru/C oxidation catalysts C1-C4 obtained in Preparation Examples 1-4, 5 g of water (the mass ratio of water to the organic phase = 1:1). After the reactor was sealed, 1 MPa of oxygen was filled to replace the residual air in the reactor. After three repetitions, 1 MPa of oxygen was filled to the reactor, and the reactor was placed on a heating furnace for heating to a reaction temperature of 100°C. The reaction was performed under stirring at a rotation rate of 700 rpm for 10 hours. After the reaction was completed, the reactor was taken out of the heating furnace and cooled to room temperature. Filtration was conducted, washing the filter cake with the same mixed solvent as the reaction solvent and finally setting the volume to 100 mL. A liquid sample was taken for high-performance liquid chromatography analysis. The reaction results are listed in Table III-1.

### Examples III-5 to III-6

The experiments were carried out according to the method of Example III-1, except that the Ru/C catalysts C5-C6 loaded on the Dali Hongye activated carbon obtained in Preparation Examples 5-6 were used as the oxidation catalyst, and the loading amount of Ru in the oxidation catalyst was 5% or 15%. The reaction results are listed in Table III-1.

### Examples III-7 to III-8

The experiments were carried out according to the method of Example III-1, except that the 10%Ru/C catalyst C1 loaded on the Dali Hongye activated carbon obtained in Preparation Example 1 was used as the oxidation catalyst, and the oxygen pressure was 2 MPa or 3 MPa. The reaction results are listed in Table III-1.

### Examples III-9 to III-10

The experiments were carried out according to the method of Example III-1, except that the 10%Ru/C catalyst C1 loaded on the Dali Hongye activated carbon obtained in Preparation Example 1 was used as the oxidation catalyst, and the reaction temperature was 120°C or 140°C. The reaction results are listed in Table III-1.

### Examples III-11 to III-15

The experiments were carried out according to the method of Example III-1, except that the catalysts C7-C11 loaded on the Dali Hongye activated carbon obtained in Preparation Examples 7-11 were used as the oxidation catalyst. The reaction results are listed in Table III-1.

**Table III-1. Reaction results of Examples III-1 to III-15**

| Example No. | Catalyst | Oxygen gas pressure /MPa | Reaction temperature /°C | HMF conversion rate /% | FDCA yield /% |
|---|---|---|---|---|---|
| III-1 | C1 | 1 | 100 | 100 | 93 |
| III-2 | C2 | 1 | 100 | 100 | 90 |
| III-3 | C3 | 1 | 100 | 90 | 70 |
| III-4 | C4 | 1 | 100 | 41 | 20 |
| III-5 | C5 | 1 | 100 | 100 | 91 |
| III-6 | C6 | 1 | 100 | 100 | 90 |
| III-7 | C1 | 2 | 100 | 100 | 90 |
| III-8 | C1 | 3 | 100 | 100 | 88 |
| III-9 | C1 | 1 | 120 | 100 | 90 |
| III-10 | C1 | 1 | 140 | 100 | 83 |
| III-11 | C7 | 1 | 100 | 100 | 92 |
| III-12 | C8 | 1 | 100 | 100 | 90 |
| III-13 | C9 | 1 | 100 | 100 | 92 |
| III-14 | C10 | 1 | 100 | 100 | 85 |
| III-15 | C11 | 1 | 100 | 100 | 91 |

It can be seen from comparing the data of Table III-1 and Table I-1 that directly using the reaction product containing 5-hydroxymethylfurfural obtained from the dehydration reaction step of the present application as the raw material for the oxidation reaction step could produce substantially comparable reaction results compared with using pure 5-hydroxymethylfurfural as the raw material. Therefore, it could be proved that the process of the present application can continuously produce 5-hydroxymethylfurfural and 2,5-furandicarboxylic acid from the fructose-based carbohydrate, and has a high raw material conversion rate and a high product yield.

### Examples III-16 to III-20

The experiments were carried out according to the method of Example III-1, except that the catalysts D1-D5 prepared in Preparation Examples 8-12 in which Pt, Pd or Au was loaded on activated carbon were used as the oxidation catalyst. The reaction results are listed in Table III-2.

**Table III-2. Reaction results of Examples III-16 to III-20**

| Example No. | Catalyst | Oxygen gas pressure /MPa | Reaction temperature /°C | HMF conversion rate /% | FDCA yield /% |
|---|---|---|---|---|---|
| III-16 | D1 | 1 | 100 | 38 | 24 |
| III-17 | D2 | 1 | 100 | 55 | 38 |
| III-18 | D3 | 1 | 100 | 80 | 58 |
| III-19 | D4 | 1 | 100 | 28 | 15 |
| III-20 | D5 | 1 | 100 | 42 | 27 |

It can be seen from the data in Table III-2, similar to the data shown in Table I-3, that all of the oxidation catalysts in which Pt, Pd, or Au was loaded on the activated carbon alone within the limited range of the present application exhibited significantly lower catalytic performance than that of the Ru/C oxidation catalyst, indicating that the Ru/C oxidation catalyst loaded on the carbon-containing material having a high specific surface area involved in the present application had a special catalytic effect on the reaction of oxidizing HMF to FDCA.

### Comparative Example III-1

The experiment was carried out according to the method of Example III-1, except that the 10%Ru/C catalyst C1 loaded on the Dali Hongye activated carbon obtained in Preparation Example 1 was used as the oxidation catalyst without adding water. After the reaction, the conversion rate of HMF was 14% and the yield of FDCA was 3%.

It can be seen from Comparative Example III-1 that without adding water, the organic solvent alone could not effectively dissolve the FDCA generated in the reaction, causing a small amount of FDCA generated in the reaction to adhere to the surface of the oxidation catalyst and poison the catalytic active site, resulting in a significant decrease in the activity of the oxidation catalyst and the yield of FDCA.

The preferable embodiments of the present application have been described in detail above. However, this application is not limited to the specific details in the above-mentioned embodiments. Within the scope of the technical concept of the present application, a variety of simple modifications can be made to the technical solutions of the present application. These simple modifications all fall within the protection scope of the present application.

In addition, it should be noted that each of the specific technical features described in the above-mentioned specific embodiments can be combined. The combinations can be made through any suitable manner without conflict. In order to avoid unnecessary repetition, various possible combinations will not be further described in the present application.

In addition, any combinations can be also made between various different embodiments of the present application, as long as they do not violate the idea of the present application; and they should also be regarded as the disclosure contents of the present application.

## Claims

1. A process for continuously producing 5-hydroxymethylfurfural and 2,5-furandicarboxylic acid, comprising the following steps:
(1) in a dual liquid phase reaction medium containing a polar organic solvent and an aqueous halogenated quaternary ammonium salt solution, in the presence of a protonic acid catalyst, subjecting a fructose-based carbohydrate to an intramolecular dehydration reaction to produce an organic phase containing 5-hydroxymethylfurfural; and
(2) adding water to the organic phase containing 5-hydroxymethylfurfural obtained in step (1), and in the presence of an oxidation catalyst and oxygen gas, subjecting the 5-hydroxymethylfurfural to an oxidation reaction to produce 2,5-furandicarboxylic acid,
wherein, the protonic acid catalyst is selected from hydrochloric acid, sulfuric acid, sulfonic acid, phosphoric acid, sulfamic acid, or combinations thereof, preferably selected from sulfamic acid, sulfuric acid and sulfonic acid;
the oxidation catalyst comprises a support and an active metal component loaded on the support, the active metal component contains a precious metal selected from Ru, Pt, Pd, Au or combinations thereof, preferably contains Ru, more preferably is Ru or a combination of Ru with one or more selected from Pt, Pd and Au.

2. The process according to claim 1, wherein the fructose-based carbohydrate in step (1) is selected from purified fructose, crude fructose, polyfructose, fructose syrup, fructose dextrose syrup, or combinations thereof.

3. The process according to claim 1 or 2, wherein the sulfonic acid is selected from methylsulfonic acid, benzene sulfonic acid or combinations thereof.

4. The process according to any one of claims 1-3, wherein in step (1) the halogenated quaternary ammonium salt is selected from a halogenated quaternary ammonium salt having a hydroxy or halogen-optionally substituted C₁₋₁₂ hydrocarbyl, preferably selected from a hydroxy or halogen-substituted C₁₋₆ hydrocarbyl trimethylammonium halide, more preferably selected from a hydroxy or halogen-optionally substituted C₁₋₄ hydrocarbyl trimethylammonium chloride and a hydroxy or halogen-optionally substituted C₁₋₄ hydrocarbyl trimethylammonium bromide, still further preferably selected from tetramethylammonium chloride, tetramethylammonium bromide, choline chloride, chlorocholine chloride, allyltrimethylammonium chloride, butyltrimethylammonium chloride, or combinations thereof, particularly preferably selected from tetramethylammonium chloride, choline chloride, chlorocholine chloride or combinations thereof.

5. The process according to any one of claims 1-4, wherein in step (1) the polar organic solvent is an aprotic polar organic solvent miscible with water, preferably selected from acetone, tetrahydrofuran, 1,4-dioxane, acetonitrile, or combinations thereof, more preferably 1,4-dioxane.

6. The process according to any one of claims 1-5, wherein the step (1) has one or more of the following features:
the mass ratio of the fructose-based carbohydrate to the reaction medium is 1:1-1000, preferably 1:2-100, more preferably 1:5-20, wherein the reaction system is a dual liquid phase reaction medium composed of the polar organic solvent and the aqueous halogenated quaternary ammonium salt solution;
the proportion of the volume of the aqueous halogenated quaternary ammonium salt solution relative to the total volume of the reaction medium is 5-50%, preferably 10-35%; based on the mass of protonic acid, the concentration of the protonic acid catalyst in the aqueous phase of the reaction medium is 0.02-0.5 g/mL, preferably 0.03-0.1 g/mL; and
the conditions for the dehydration reaction include: reaction temperature of 80-200°C, preferably 100-150°C; reaction time of 0.1-12 hours, preferably 0.1-1 hour.

7. The process according to any one of claims 1-6, wherein the oxidation catalyst in step (2) has one or more of the following features:
the active metal component is Ru or a combination of Ru with one or more selected from Pt, Pd and Au, based on the total mass of the active metal component, the mass content of Ru is 5-100%, preferably 20-100%, more preferably 40-100%, further preferably 50-100%; based on the total mass of the support, the content of the active metal component in the oxidation catalyst is 0.25-30%, preferably 1-20%, more preferably 5-15%, particularly preferably the content of Ru is 5-15%;
the support is selected from carbon-containing materials, preferably selected from activated carbon, carbon nanotube, graphene, graphene oxide, or combinations thereof, more preferably activated carbon, particularly preferably activated carbon having a specific surface area of 1000-2000 m²/g, preferably 1000-1500 m²/g.

8. The process according to any one of claims 1-7, wherein step (2) has one or more of the following features:
the mass ratio of the organic phase containing 5-hydroxymethylfurfural to water is 5:1-0.5:1, preferably 3:1-1:1;
the oxidation catalyst is used in such an amount that the molar ratio of the active metal component in the oxidation catalyst to the 5-hydroxymethyl furfural is 1:1-1000, preferably 1:5-500, more preferably 1:5-250;
the oxidation reaction is carried out under an atmosphere of oxygen gas, an atmosphere of air, or a mixed atmosphere of oxygen gas and nitrogen gas; and
the conditions for the oxidation reaction comprise: reaction temperature of 50-170°C, preferably 90-150°C, and oxygen gas partial pressure of 0.2-4 MPa, preferably 1-3 MPa.

9. A process for producing 2,5-furandicarboxylic acid, comprising the following steps:
in a mixed solvent containing an organic solvent and water, in the presence of an oxidation catalyst and oxygen gas, subjecting 5-hydroxymethylfurfural to an oxidation reaction to produce 2,5-furandicarboxylic acid;
wherein, the organic solvent is an aprotic polar organic solvent miscible with water, preferably selected from acetone, tetrahydrofuran, 1,4-dioxane, acetonitrile, dimethyl sulfoxide, or combinations thereof, more preferably 1,4-dioxane;
the oxidation catalyst comprises a support and an active metal component loaded on the support, wherein the support is an activated carbon having a specific surface area of 1000-2000 m²/g, preferably 1000-1500 m²/g, and the active metal component contains Ru, preferably is Ru or a combination of Ru with one or more selected from Pt, Pd and Au.

10. The process according to claim 9, wherein the active metal component is Ru or a combination of Ru with one or more selected from Pt, Pd and Au, based on the total mass of the active metal component, the mass content of Ru is 40-100%, preferably 50-100%, more preferably 60-100%;
further preferably, based on the total mass of the support, the content of the active metal component in the oxidation catalyst is 0.25-30%, preferably 1-20%, more preferably 5-15%, particularly preferably the content of Ru is 5-15%.

11. The process according to claim 9 or 10, which has one or more of the following features:
the mixed solvent is composed of the organic solvent and water, wherein the mass ratio of the organic solvent to water is preferably 5:1-0.5:1, more preferably 3:1-1:1;
in a solution formed from the 5-hydroxymethylfurfural and the mixed solvent, the mass percentage of the 5-hydroxymethylfurfural is 0.1-30%, preferably 0.5-20%, more preferably 1-10%;
the oxidation catalyst is used in such an amount that the molar ratio of the active metal component in the oxidation catalyst to the 5-hydroxymethyl furfural is 1:1-1000, preferably 1:5-500, more preferably 1:5-250;
the conditions for the oxidation reaction comprise: reaction temperature of 50-170°C, preferably 90-150°C, and oxygen gas partial pressure of 0.2-4 MPa, preferably 1-3 MPa; and
the oxidation reaction is carried out under an atmosphere of oxygen gas, an atmosphere of air, or a mixed atmosphere of oxygen gas and nitrogen gas.

12. A reaction system for continuously producing 5-hydroxymethylfurfural and 2,5-furandicarboxylic acid, which comprises a dehydration reaction unit, a phase separation unit, and an oxidation reaction unit, wherein the dehydration reaction unit has an inlet and an outlet, the phase separation unit has an inlet, an aqueous phase outlet and an organic phase outlet, the oxidation reaction unit has an inlet and an outlet, the outlet of the dehydration reaction unit is communicated with the inlet of the phase separation unit, the aqueous phase outlet of the phase separation unit is communicated with the inlet of the dehydration reaction unit, and the organic phase outlet of the phase separation unit is communicated with the inlet of the oxidation reaction unit,
wherein a fructose-based carbohydrate is subjected to a dehydration reaction in a dual liquid phase reaction medium containing a polar organic solvent and an aqueous halogenated quaternary ammonium salt solution in the presence of a protonic acid catalyst in the dehydration reaction unit to produce a two-phase stream containing 5-hydroxymethylfurfural, the two-phase stream is subjected to a phase separation in the phase separation unit to produce an aqueous phase containing the acid catalyst and an organic phase containing 5-hydroxymethylfurfural, the aqueous phase containing the acid catalyst is fed back to the dehydration reaction unit, and the organic phase containing 5-hydroxymethylfurfural is subjected to an oxidation reaction in the oxidation reaction unit to produce a stream containing 2,5-furandicarboxylic acid;
preferably, the dehydration reaction unit includes a dehydration reactor selected from a slurry bed, a bubbling bed, a shell and tube reactor or combinations thereof;
preferably, the oxidation reaction unit includes an oxidation reactor selected from a slurry bed, a fixed bed, a bubbling bed, or combinations thereof; and
preferably, the phase separation unit includes a phase separation tank.

13. The reaction system according to claim 12, further comprising a purification unit and a drying unit, wherein the stream containing 2,5-furandicarboxylic acid from the oxidation reaction unit is treated in the purification unit and the drying unit to obtain a product of 2,5-furandicarboxylic acid;
preferably, the purification unit includes an equipment selected from an adsorption column, a vacuum distillation column, a molecular distiller, a fluidized bed, a tubular continuous crystallizer, a precipitation crystallizer or combinations thereof;
preferably, the drying unit includes a dryer selected from a spray dryer, a fluidized bed dryer, a pneumatic dryer, a box-type dryer or combinations thereof.

14. The reaction system according to claim 12 or 13, further comprising a decolorization and purification unit and a freeze-drying unit, wherein a part of the organic phase containing 5-hydroxymethylfurfural from the phase separation unit is treated in the decolorization and purification unit and the freeze-drying unit to obtain a product of 5-hydroxymethylfurfural,
preferably, the decolorization and purification unit comprises an activated carbon adsorption column, a vacuum distillation column and a crystallizer; and
preferably, the freeze-drying unit comprises a freeze-dryer.
